# EUROPÄISCHE PATENTSCHRIFT

(11) **EP 1 594 612 B1**
(45) Veröffentlichungstag und Bekanntmachung des Hinweises auf die Patenterteilung: **07.06.2006**
(21) Anmeldenummer: 04710332.0
(22) Anmeldetag: 12.02.2004
(51) Int. Cl.: B01L 1/00, G01N 33/15, G01N 35/04

(54) **VORRICHTUNG ZUR QUALITÄTSKONTROLLE FESTER, PHARMAZEUTISCHER ERZEUGNISSE**
QUALITY CONTROL DEVICE FOR SOLID, PHARMACEUTICAL PRODUCTS
INSTALLATION POUR CONTROLER LA QUALITE DE PRODUITS PHARMACEUTIQUES SOLIDES

(30) Priorität: 12.02.2003 DE 10305966; 07.04.2003 DE 10316024
(43) Veröffentlichungstag der Anmeldung: 16.11.2005
(73) Patentinhaber: Krämer, Thilo, 64291 Darmstadt (DE)
(72) Erfinder: Krämer, Thilo, 64291 Darmstadt (DE)
(74) Vertreter: Mierswa, Klaus
(86) Internationale Anmeldenummer: PCT/DE2004/000253
(87) Internationale Veröffentlichungsnummer: WO 2004/071659

(56) Entgegenhaltungen:
- CH-A- 690 962
- DE-A- 10 024 598
- DE-B- 1 008 934
- DE-C- 3 235 927
- US-A- 3 041 957
- US-A- 3 874 754

## Beschreibung

### Technisches Gebiet:

Die Erfindung betrifft eine Vorrichtung zur Qualitätskontrolle fester, pharmazeutischer Erzeugnisse, wie Tabletten, Pillen, Oblongs oder dergleichen, die wenigstens eine Prüfeinrichtung und wenigstens eine Transporteinrichtung sowie wenigstens einen Sensor zur Prüfung und Erfassung eines oder mehrerer Parameter des einzelnen pharmazeutischen Erzeugnisses aufweist, wie Waage und/oder Höhenmesseinrichtung und/oder Kraftmesseinrichtung zur Messung des Gewichts und/oder der Dicke und/oder der Berstkraft des Erzeugnisses, nach der Gattung des Hauptanspruchs.

### Stand der Technik:

Es sind Vorrichtungen zur Qualitätskontrolle fester pharmazeutischer Erzeugnisse, wie beispielsweise Tabletten, Pillen, Oblongs oder dergleichen bekannt, bei denen Erzeugnisse einzeln verschiedenen Prüfeinrichtungen, beispielsweise für Gewicht, Härte, Abmessungen, Abriebeigenschaften oder dergleichen, zugeführt werden. Bei einer bekannten Vorrichtung zur Qualitätskontrolle gattungsgemäßer Art, welche einen durch eine nach oben hin abnehmbare Schutzhaube gebildeten Prüfraum aufweist, werden homogene, feste pharmazeutische Erzeugnisse in einen Speicherbehälter geschüttet und gelangen von diesem aus zu einer Vereinzelungsrinne, an deren Ende die Erzeugnisse von einer Ladeeinrichtung einzeln erfasst und in je eine zum Prüfraumboden hin offene Aufnahmekammer eines Transportsterns von der Ladeeinrichtung abgelegt wird. Der Transportstern ist um seine vertikale Achse rotierend in einem geringem Abstand parallel über dem Prüfraumboden angeordnet, so dass das in der Aufnahmekammer liegende Erzeugnis nicht in den Spalt zwischen Transportstern und Prüfraumboden gelangen kann. In der Aufnahmekammer des Transportsterns wird das Erzeugnis beispielsweise zur Bestimmung seines Gewichts über einen Waagentisch einer bündig im Prüfraumboden angeordnete Waage geführt und anschließend mittels einer Auswurföffnung im Prüfraumboden aus der Aufnahmekammer entfernt, die radial vom Mittelpunkt des Transportsterns beabstandet angeordnet ist, so dass durch die Rotation des Transportsterns die Aufnahmekammer nach der Waage über die Auswurföffnung bewegt wird und das in der Aufnahmekammer befindliche Erzeugnis durch die Auswurföffnung hindurch in einen Kanal fällt, der über einem Sammelbehälter endet, so dass die geprüften Erzeugnisse in diesem Sammelbehälter beispielsweise für nachfolgende Test oder Kontrollen gesammelt werden können.

Typischerweise wird eine solche Vorrichtung zur Qualitätskontrolle fester pharmazeutischer Erzeugnisse für nacheinander stattfindende Kontrollen an verschiedenen pharmazeutischen Erzeugnissen verwendet. Hierfür werden aus der laufenden Produktion regelmäßig Erzeugnisse entnommen und so lange gesammelt, bis eine ausreichend große Anzahl für einen Durchlauf in der Qualitätskontrolle zur Verfügung steht.

Um beispielsweise nachfolgende Qualitätstests, wie beispielsweise eine Wirkstoffanalyse, nicht durch Staub oder sonstige Reste von zuvor in der selben Vorrichtung geprüfte Erzeugnisse zu verfälschen, muss vor jeder Prüfung eines anderen Erzeugnisses ein sehr gründliches Reinigen der Prüfeinrichtung möglich sein. Dies ist insbesondere bei Kombinationsgeräten, bei denen neben dem Wiegen beispielsweise auch Härtetests, Dickenmessungen oder dergleichen durchführbar sind, von Bedeutung, wenn an demselben Gerät nach Härteprüfungen, bei denen die Erzeugnisse zerstört werden, Qualitätskontrollen anderer Erzeugnisse stattfinden.

Ein Reinigen solcher Vorrichtungen ist zeitintensiv, da empfindliche Bauteile wie beispielsweise Waage, Steuerung der Vereinzelungsrinne, Steuerung der Ladeeinrichtung, Speichereinrichtung oder dergleichen zur Messdatenerfassung und -aufzeichnung und zugehörige Sensoren im zu reinigenden Prufraum angeordnet sind oder zu diesem hin Öffnungen aufwiesen, so dass das Bedienpersonal sorgfältig vorgehen muss, um Beschädigungen an diesen Bauteilen insbesondere durch Reinigungslösungen, Reinigungshilfsmittel wie Tücher oder dergleichen zu vermeiden.

Bekannte Vorrichtungen zur Qualitätskontrolle der gattungsgemäßen Art weisen darüber hinaus den Nachteil auf, dass Zuführung, Vereinzelung, Prüfung und Sammeln der Erzeugnisse, sowie die Aufzeichnung der gewonnenen Prüfdaten in einem Gerät an dessen Aufstellungsort erfolgt.

Durch die DE 100 24 598 A1 ist eine Vorrichtung zur automatischen Qualitätskontrolle von Prüflingen, wie Tablets, Tabletten, Pillen oder Dragées, zur Bestimmung von mechanisch-physikalischen Eigenschaften derselben, wie Gewicht, Abmessungen, Zerfallszeit in einem Medium sowie Härte, bekannt geworden. Die Vorrichtung besteht aus einer Zuführeinrichtung und einem Transportstern mit peripher angeordneten Aufnahmekammern für je einen Prüfling sowie einem Härtetester, gegebenenfalls einer Waage sowie einer Einrichtung zur Bestimmung der Abmessungen des Prüflings, wobei an einen Abgang des Transportsterns zur Übernahme des Prüflings ein Linearförderer angeschlossen ist, an den sich der Härtetester anschließt und der Linearförderer tangential an dem Transportstern angeordnet ist. Die Vorrichtung weist eine Datenverarbeitungseinrichtung zur Aufnahme und Verarbeitung sämtlicher Daten mit einem Hauptprozessor und einem Zusatzprozessor auf, der mit dem Hauptprozessor zur Koordination der Bewegung des Härtetesters mit dem laufenden Transportstern zusammenarbeitet.

Die Dokumente DE 32 35 927 und CH 690 962 offenbaren Analysevorrichtungen, die sich in einer geschützten Kabine befinden. Die Kabinen aus diesen Dokumenten sind als feste Anordnungen ausgebildet.

Dokument US 3 041 957 offenbart eine abnehmbare Schutzhaube, die als Spritzschutz in z.B. Laborvorführungen verwendet wird.

### Technische Aufgabe der Erfindung:

Der Erfindung liegt daher die Aufgabe zugrunde, eine Vorrichtung zur Qualitätskontrolle fester, pharmazeutischer Erzeugnisse zu entwickeln, die leicht und schnell und insbesondere gründlich zu reinigen ist, um die Umrüstzeiten bei der Umstellung auf andere Erzeugnisse zu verkürzen, und die unabhängig von ihrem Aufstellungsort eine Auswertung der gewonnenen Prüfdaten an einem beliebigen anderen Ort zulässt. Zweck der Erfindung ist somit die Schaffung einer Vorrichtung, welche gründlich und schnell sowie automatisch zu reinigen ist ebenso wie sie eine Datenweitergabe entfernt möglich machen soll.

### Offenbarung der Erfindung und deren Vorteile:

Die Aufgabe wird erfindungsgemäß gelöst durch eine Vorrichtung zur Qualitätskontrolle fester pharmazeutischer Erzeugnisse, wie Tabletten, Pillen, Oblongs oder dergleichen, mit wenigstens einer Prüfeinrichtung, welche mindestens einem Sensor zur Prüfung und Erfassung eines oder mehrerer Parameter des einzelnen pharmazeutischen Erzeugnisses aufweist, wie Waage und/oder Höhenmesseinrichtung und/oder Kraftmesseinrichtung zur Messung der Berstkraft des Erzeugnisses, sowie mit einer Transporteinrichtung für die Erzeugnisse, dadurch gekennzeichnet, dass die Vorrichtung ein Gehäuse und einen darüber angeordneten, gegenüber der Umgebung spritzwasserdicht oder wasser- und staubdicht verschließbaren Prüfraum aufweist, welcher durch einen Boden und eine darauf aufsetzbare und abnehmbare Schutzhaube gebildet ist und in welchem sich die Prüfeinrichtung sowie die Transporteinrichtung befinden.

Gemäß einer Variante der Erfindung ist die Schutzhaube in lösbarer Weise spritzwasserdicht oder wasser- und staubdicht mit dem Boden des Prüfraumes verbindbar.

Gemäß einer weiteren Variante der Erfindung ist die Schutzhaube spritzwasserdicht oder wasser- und staubdicht peripher auf den Boden des Prüfraumes aufpressbar und verriegelbar aufgesetzt, wobei die Schutzhaube in ihrer dem Boden des Prüfraums zugewandten Stirnfläche eine umlaufende spritzwasserdichte oder wasser- und staubdichte Dichtung aufweist

Gemäß einer weiteren Variante der Erfindung ist das Gehäuse ebenfalls spritzwasserdicht oder wasser- und staubdicht verschließbar.

Gemäß einer weiteren Variante der Erfindung sind in dem Gehäuse Einrichtungen zum Antrieb, zur Steuerung und zur Versorgung der Prüfeinrichtung und der Transporteinrichtung bzw. der Prüfeinrichtungen und Transporteinrichtungen angeordnet.

Gemäß einer weiteren Variante der Erfindung sind sämtliche in den Prüfraum hinein- oder aus demselben herausführenden führenden Leitungen und/oder Antriebswellen spritzwasserdicht oder wasser- und staubdicht durch die Schutzhaube oder den Boden durchgeführt.

Gemäß einer weiteren Variante der Erfindung weist der Boden mindestens einen Durchbruch, insbesondere zur Durchführung von Leitungen oder einer Antriebswelle, auf, welcher mittels einer spritzwasserdichten oder einer wasser- und staubdichten Dichtung abgedichtet ist.

Gemäß einer weiteren Variante der Erfindung sind die Dichtungen aufblasbare O-Ringdichtungen.

Gemäß einer weiteren Variante der Erfindung sind zur Gewährleistung der Spritzwasserdichtheit oder Wasserdichtheit des Prüfraumes sämtliche Zulauf- oder Zuführöffnungen in den Prüfraum und sämtliche Ablauf- oder Abführöffnungen aus dem Prüfraum heraus hermetisch verschließbar.

Gemäß einer weiteren Variante der Erfindung ist der Boden des Prüfraumes zugleich eine Wandung des Gehäuses.

Gemäß einer weiteren Variante der Erfindung sind innerhalb des Gehäuses Einrichtungen zum Antrieb, zur Steuerung und zur Versorgung der Prüfeinrichtungen und Transporteinrichtungen, wie Antriebsaggregate, Elektromotoren, elektrische Steuerungen, Signalverstärker, sowie elektrische und/oder optische und/oder mechanische Anschlüsse für die Energieversorgung der Prüfeinrichtung, angeordnet.

Gemäß einer weiteren Variante der Erfindung ist an dem Gehäuse, vorzugsweise an dessen Vorderwandung, eine von außerhalb des Gehäuses zugängliche Schnittstelle angeordnet, in welche eine Mehrzahl von aus dem Gehäuse heraus- oder in dasselbe hineinführenden elektrischen Leitungen mündet und welche zum elektrischen Anschluss der Vorrichtung und/oder zur Übertragung von elektrischen Signalen und/oder Daten zwischen der Vorrichtung und der Außenwelt dient.

Gemäß einer weiteren Variante der Erfindung ist an dem Gehäuse eine Schwenktür so angeordnet, dass sich zwischen dem Gehäuse und der Schwenktür ein Vorraum befindet.

Gemäß einer weiteren Variante der Erfindung umfasst die Vorrichtung folgende weiteren Bestandteile:
- eine innerhalb des Prüfraumes als Teil der Transporteinrichtung angeordnete Vereinzelungseinrichtung, welcher die pharmazeutischen Erzeugnisse aus einem Speicherbehälter zuführbar sind,
- eine über dem Boden des Prüfraumes als weiterer Teil der Transporteinrichtung angeordnete Fördereinrichtung, welche mindestens eine zum Boden hin offene Aufnahmezelle aufweist, in welche jeweils ein Erzeugnis aus der Vereinzelungseinrichtung einführbar ist, wobei das in der Aufnahmezelle aufgenommene Erzeugnis der Prüfeinrichtung zuführbar ist,
- eine Auswurföffnung, welcher das Erzeugnis nach Prüfung durch die Prüfeinrichtung in Fortsetzung des Transportes durch die Fördereinrichtung zuführbar ist, wobei das Erzeugnis nach Erreichen der Auswurföffnung durch diese hindurchfällt und auf diese Weise aus der Aufnahmezelle entfernt wird,
- sowie einen unterhalb der Auswurföffnung angeordneten Sammelbehälter zur Aufnahme der geprüften Erzeugnisse oder deren Teile, nachdem diese durch die Auswurföffnung hindurchgefallen sind.

Gemäß einer weiteren Variante der Erfindung ist die Transporteinrichtung ein parallel zum Boden angeordneter, um eine vertikale Achse rotierbarer Transportstern , wobei die Aufnahmezelle im Bereich des Außenumfangs des Transportsterns angeordnet ist und wobei die Prüfeinrichtung sowie die Auswurföffnung so angeordnet sind, dass ein in der Aufnahmezelle aufgenommenes Erzeugnis bei einer Rotation des Transportsterns in dessen Drehrichtung nacheinander zunächst die Prüfeinrichtung und anschließend die Auswurföffnung erreicht.

Gemäß einer weiteren Variante der Erfindung ist die Auswurföffnung ein Loch im Boden des Prüfraumes, wobei sich der Sammelbehälter in dem Vorraum befindet.

Gemäß einer weiteren Variante der Erfindung weist der Boden des Prüfraums um die Auswurföffnung einen Süllrand auf.

Gemäß einer weiteren Variante der Erfindung ist die Prüfeinrichtung eine Waage mit einem Waagentisch und einer Kraftmessdose.

Gemäß einer weiteren Variante der Erfindung ist das Innere des Gehäuses mit einem Überdruck gegenüber der Umgebung und/oder dem Prüfraum beaufschlagbar.

Gemäß einer weiteren Variante der Erfindung ist der Prüfraum mit einem Unterdruck gegenüber der Umgebung beaufschlagbar.

Gemäß einer weiteren Variante der Erfindung ist das Gehäuse statt unter dem Boden des Prüfraumes neben demselben angeordnet.

Gemäß einer weiteren Variante der Erfindung weist die Vorrichtung Rollen oder Räder auf, mittels welchen die Vorrichtung verfahrbar ist.

Gemäß einer weiteren Variante der Erfindung besteht die Vorderwandung des Gehäuses aus einem Rahmen und einer auf diesen wasserdicht aufgesetzten abnehmbaren Frontplatte.

Gemäß einer Variante weist die erfindungsgemäße Vorrichtung zur Qualitätskontrolle fester, pharmazeutischer Erzeugnisse, wie Tabletten, Pillen, Oblongs oder dergleichen wenigstens eine Prüfeinrichtung mit wenigstens einem Sensor zur Prüfung und Erfassung eines oder mehrerer Parameter der pharmazeutischen Erzeugnisse auf, wie eine Waage und/oder eine Höhenmesseinrichtung und/oder eine Kraftmesseinrichtung zur Messung des Gewichts und/oder der Dicke und/oder der Berstkraft des einzelnen Erzeugnisses, wobei die Vorrichtung aus einem Gehäuse mit einem darüber angeordneten, geschlossenen Prüfraum besteht, welcher durch einen Boden und eine darauf aufsetzbare, bewegliche Schutzhaube gebildet ist, in welchem Prüfraum sich die Prüfeinrichtungen befinden, wobei Antriebe und gegebenenfalls Versorgungseinrichtungen der im Prüfraum angeordneten Prüfeinrichtungen und Transporteinrichtungen in dem gegenüber dem Prüfraum und der Umgebung mindestens spritzwasser- und staubdicht gekapselten separaten Gehäuse angeordnet sind, und wobei wenigstens Teile der Zuleitungen zu den Antrieben und den Versorgungseinrichtungen für die Energieversorgung der Prüfeinrichtung innerhalb des Gehäuses in ebenfalls mindestens spritzwasser- und staubdicht gekapselter Weise nach außerhalb des Prüfraumes geführt sind.

Gemäß einer weiteren Variante der Erfindung sind die Zuleitungen zu den Antrieben und gegebenenfalls Versorgungseinrichtungen für die Energieversorgung der Prüfeinrichtungen und Transporteinrichtungen durch den Prüfraumboden in einen unterhalb des geschlossenen Prüfraumes befindlichen, durch das Gehäuse gebildeten Aufnahmeraum geführt, so dass die Vorrichtung durch den Prüfraumboden in den Prüfraum und in den Aufnahmeraum des Gehäuses geteilt ist, wobei das den Aufnahmeraum umschließende Gehäuse gleichermaßen durch eine Tür oder dergleichen wasser- und staubdicht verschließbar ist und innerhalb des Aufnahmeraumes Einrichtungen zum Antrieb, zur Steuerung und zur Versorgung der Prüfeinrichtungen und Transporteinrichtungen wie Antriebsaggregate, Elektromotoren, elektrische Steuerungen, Signalverstärker, sowie elektrische und/oder optische und/oder mechanische Anschlüsse für die Energieversorgung der Prüfeinrichtung, angeordnet sind.

Gemäß einer weiteren Variante der Erfindung weist das Gehäuse des Aufnahmeraumes mindestens eine von außerhalb des Gehäuses zugängliche Schnittstelle auf, vorzugsweise in der vorderen Wandung des Gehäuses, zum Anschluss von elektrischen und/oder optischen und/oder mechanischen Anschlüssen für die Energieversorgung der Prüfeinrichtungen und zum Anschluss eines Computers oder dergleichen für die Funktionsüberwachung und die Erfassung und Weiterleitung von Prüfdaten der Sensoren, so dass eine Steuerung der Vorrichtung wie auch eine Auswertung der Prüfdaten unabhängig vom Aufstellungsort der Vorrichtung durchführbar ist.

Gemäß einer weiteren Variante der Erfindung ist die Schutzhaube ebenfalls wasser- und staubdicht peripher auf den Boden des Prüfraumes aufpressbar und verriegelbar aufgesetzt.

Gemäß einer weiteren Variante der Erfindung weist die Schutzhaube in ihrer dem Boden des Prüfraums zugewandten Stirnfläche eine in der Stirnfläche umlaufende Dichtung auf.

Gemäß einer weiteren Variante der Erfindung überragen der Boden des Prüfraumes sowie die Schutzhaube das Gehäuse des Aufnahmeraumes wenigstens an einer Seite, vorzugsweise die Vorderseite des Gehäuses, wobei an dem Gehäuse eine Schwenktür angeordnet ist, welche unterhalb des Bodens und zwischen der der Schwenktür gegenüber liegenden Vorderwandung des Gehäuses einen weiteren Raum, nämlich den Vorraum, abtrennt, in welchen die von außen zugängliche Schnittstelle des Gehäuses mündet.

Gemäß einer weiteren Variante der Erfindung weist die Vorrichtung eine innerhalb des Prüfraumes angeordnete Vereinzelungseinrichtung auf, welcher die pharmazeutischen Erzeugnisse aus einem Speicherbehälter zuführbar sind, wobei die Vorrichtung eine über dem Boden des Prüfraumes angeordnete Transporteinrichtung aufweist, welche mindestens eine zum Boden hin offenen Aufnahmezelle aufweist, in welche jeweils ein Erzeugnis aus der Vereinzelungseinrichtung zuführbar ist, wobei das Erzeugnis in der Aufnahmezelle mindestens einer vorzugsweise unter der Transporteinrichtung angeordneten Prüfeinrichtung zur Prüfung des Erzeugnisses auf Gewicht und/oder Abmessungen und/oder Härte und/oder Abriebeigenschaften zugeführt wird, und mittels einer nach der Prüfeinrichtung im Boden angeordneten Auswurföffnung, in welche das Erzeugnis nach Prüfung und Fortsetzung des Transportweges hineinfällt, aus der Aufnahmezelle entfernt wird, wobei unterhalb der Auswurföffnung ein Sammelbehälter zur Aufnahme der geprüften Erzeugnisse oder deren Teile angeordnet ist.

Gemäß einer weiteren Variante der Erfindung ist die Transporteinrichtung ein um eine vertikale Achse rotierbar angeordneter Transportstern, an dessen Außenumfang sich mindestens eine Aufnahmezelle befindet und in dessen Drehrichtung sich zunächst die Prüfeinrichtung befindet und anschließend in der Drehrichtung die Auswurföffnung angeordnet ist.

Gemäß einer weiteren Variante der Erfindung befindet sich die Auswurföffnung sich in dem Teil des Bodens des Prüfraumes, welcher das Gehäuse des Aufnahmeraumes überragt, wobei sich der unterhalb der Auswurföffnung befindliche Sammelbehälter in dem weiteren Freiraum befindet, welcher durch die Schwenktür abgetrennt ist.

Gemäß einer weiteren Variante der Erfindung ist die Prüfeinrichtung eine Waage mit einem Waagentisch und einer Kraftmessdose, wobei der Waagentisch über dem Boden des Prüfraums angeordnet ist und der Boden unter dem Waagentisch einen Süllrand aufweist, und der Waagentisch eine Ausnehmung zur Aufnahme des Süllrandes besitzt, so dass durch das Zusammenwirken von Süllrand und Ausnehmung am Waagentisch eine mindestens spritzwasserdichte Labyrinthdichtung entsteht.

Gemäß einer weiteren Variante der Erfindung ist der Transportstern parallel zum Boden des Prüfraums um die Höhe des Waagentisches über dem Boden beabstandet angeordnet, wobei auf den Boden mindestens eine Platte auflegbar ist, die in ihrer Dicke der Höhe des Waagentisches über dem Boden des Prüfraums entspricht und wobei die Platte Ausnehmungen für die Auswurföffnung, den Waagentisch, die Durchführung der Antriebswellen des Transportsterns und dergleichen aufweist.

Gemäß einer weiteren Variante der Erfindung ist die Kraftmessdose der Waage direkt unter dem Waagentisch anordbar.

Gemäß einer weiteren Variante der Erfindung weist der Boden des Prüfraums einen Süllrand um die Auswurföffnung auf.

Gemäß einer weiteren Variante der Erfindung ist die Auswurföffnung durch einen Pfropfen verschließbar.

Gemäß einer weiteren Variante der Erfindung sind in bzw. an der Wandung des Gehäuses innerhalb des weiteren, von der Schwenktür abgetrennten Raumes, d.h. des Vorraumes, elektrische und/oder optische Anschlüsse zum Anschluss eines Computers oder dergleichen für die Funktionsüberwachung und die Erfassung und Weiterleitung der Prüfdaten angeordnet, wobei der Computer in diesem Raum anordbar ist.

Gemäß einer weiteren Variante der Erfindung ist die Schutzhaube nur nach dem Öffnen der Schwenktür am Boden des Prüfraumes öffenbar oder verriegelbar.

Gemäß einer weiteren Variante der Erfindung ist in dem den Aufnahmeraum bildenden Gehäuse ein konstanter Überdruck gegenüber der Umgebung und/oder dem Prüfraum einbringbar.

Gemäß einer weiteren Variante der Erfindung ist in dem Prüfraum ein konstanter Unterdruck gegenüber der Umgebung einbringbar.

Gemäß einer weiteren Variante der Erfindung besteht die Vorrichtung aus einem Gehäuse mit einem darüber angeordneten, verschließbaren Prüfraum, der durch einen Boden und eine darauf aufsetzbare, bewegliche Schutzhaube gebildet ist, in welchem Prüfraum sich die Prüfeinrichtung sowie die Transporteinrichtung befinden, wobei Antriebe und gegebenenfalls Versorgungseinrichtungen für die im Prüfraum angeordneten Prüf- und Transporteinrichtung in dem gegenüber dem Prüfraum - gegebenenfalls auch gegenüber der übrigen Umgebung - wasserdicht und staubdicht gekapselten separaten Gehäuse angeordnet sind.

In einer vorteilhaften Ausgestaltung der Erfindung sind wenigstens Teile der Zuleitungen zu den Antrieben und den Versorgungseinrichtungen für die Energieversorgung der Prüfeinrichtung sowie der Transporteinrichtung innerhalb des Gehäuses in wasserdicht und staubdicht gekapselter Weise nach außerhalb des Prüfraumes geführt. In weiterer Ausgestaltung der Erfindung sind sämtliche Durchbrüche durch den Boden zur Durchführung von Zuleitungen sowie zur Aufnahme von Prüfeinrichtungen mit wasserdichten Dichtungen ausgestattet, welche zum Beispiel aufblasbare O-Ringdichtungen sein können.

Die erfindungsgemäße Vorrichtung zur Qualitätskontrolle fester, pharmazeutischer Erzeugnisse weist gegenüber dem Stand der Technik den Vorteil auf, dass die Antriebe und Versorgungseinrichtungen sowie die Signalverstärker aller in einem durch eine Schutzhaube schließbaren Prüfraum angeordneten Einrichtungen zur Vereinzelung, Transport und Prüfung der Erzeugnisse in gegenüber dem Prüfraum spritzwasserdicht oder wasserdicht und staubdicht verschließbaren Gehäusen untergebracht sind, so dass beispielsweise Reinigungsarbeiten mit Hilfe von Reinigungslösungen, Druckreinigern oder dergleichen schnell und einfach durchführbar sind, wobei die Leitungen für die Energieversorgung der Antriebe, Versorgungseinrichtungen und Signalverstärker von den geschlossenen Gehäusen aus nach außerhalb des Prüfraumes in spritzwasserdicht oder wasserdicht abgedichteter Weise geführt werden.

Eine vorteilhafte Ausgestaltung der Erfindung sieht vor, dass die Zuleitungen zu den Antrieben und gegebenenfalls Versorgungseinrichtungen für die Energieversorgung der Prüfeinrichtungen und Transporteinrichtungen durch den Prüfraumboden in einen unterhalb des geschlossenen Prüfraumes befindlichen, durch das Gehäuse gebildeten Aufnahmeraum geführt sind, so dass die Vorrichtung durch den Prüfraumboden einerseits in den Prüfraum und andererseits in den Aufnahmeraum des Gehäuses geteilt ist, wobei das den Aufnahmeraum umschließende Gehäuse durch eine Tür oder dergleichen wasser- und staubdicht verschließbar ist und innerhalb des Aufnahmeraumes Einrichtungen zum Antrieb, zur Steuerung und zur Versorgung der Prüfeinrichtungen und Transporteinrichtungen wie Antriebsaggregate, Elektromotoren, elektrische Steuerungen, Signalverstärker, sowie elektrische und/oder optische und/oder mechanische Anschlüsse für die Energieversorgung der Prüfeinrichtung, angeordnet sind.

Damit kann eine vollständige, gründliche und insbesondere automatische Reinigung der Vorrichtung durchgeführt werden. Nach dem bestimmungsgemäßen Gebrauch der Vorrichtung werden hierzu sämtliche Zuführ- oder Zulauf öffnungen sowie Abfuhr- oder Ablauföffnungen hermetisch wasserdicht geschlossen, wie auch sämtliche Durchbrüche durch den Boden des geschlossenen Prüfraumes hermetisch wasserdicht verschlossen werden, zum Beispiel durch O-Ringe, welche in besonderer Ausgestaltung auch aufblasbar sein können. Der Prüfraum wird somit so wasserdicht wie ein Aquarium gemacht. Nunmehr wird in den Prüfraum Wasser eingefüllt und die Antriebe der Vorrichtung werden eingeschaltet, so dass sich sämtliche rotierenden oder hin- und hergehenden Teile innerhalb des geschlossenen Prüfraumes innerhalb des Wasserbades bewegen und dadurch eine automatische Reinigung durchführen. Anschließend kann die Vorrichtung, falls erforderlich, in einen Reinstraum verbracht werden.

In einer weiteren vorteilhaften Ausgestaltung der Erfindung weist das Gehäuse des Aufnahmeraumes, vorzugsweise innerhalb der vorderen Außenwand des Gehäuses, mindestens eine von außerhalb des Gehäuses zugängliche Schnittstelle zum Anschluss von elektrischen und/oder optischen und/ oder mechanischen Anschlüssen für die Energieversorgung der Prüfeinrichtungen und zum Anschluss eines Computers oder dergleichen für die Funktionsüberwachung und die Erfassung und Weiterleitung von Prüfdaten der Sensoren auf, so dass eine Steuerung und Funktionsüberwachung der Vorrichtung wie auch eine Auswertung der Prüfdaten unabhängig vom Aufstellungsort der Vorrichtung und von eventuell notwendigen Reinigungsarbeiten durchführbar ist. Durch die Schnittstelle werden Durchführungen von Kabeln oder dergleichen durch die Gehäusewand verhindert, so dass die wasser- und staubdichte Ausführung des Gehäuses gewährleistet ist. Somit überragen der Boden des Prüfraumes sowie die Schutzhaube das Gehäuse des Aufnahmeraumes wenigstens an einer Seite, vorzugsweise an der Vorderseite des Gehäuses, wobei an dem Gehäuse eine Schwenktür angeordnet ist, welche unterhalb des Bodens des Prüfraumes und zwischen der der Schwenktür gegenüber liegenden Vorderseite des Gehäuses einen weiteren Raum abtrennt, in welchen die von außen zugängliche Schnittstelle des Gehäuses mündet.

Eine vorteilhafte Ausgestaltung der Erfindung sieht vor, dass die Schutzhaube den Prüfraum gegenüber der Umgebung wasser- und staubdicht abschließt, um das Austreten von Abriebstaub oder dergleichen aus dem Prüfraum zu verhindern und dass die Schutzhaube verriegelt und auf den Boden des Prüfraumes gepresst werden kann.

In weiterer Ausgestaltung der Vorrichtung weist die Schutzhaube an ihrer dem Boden des Prüfraums zugewandten Stirnseite eine umlaufende Dichtung auf, so dass durch das Anpressen der Schutzhaube eine hohe Dichtigkeit erreicht werden kann, wodurch Schmutz oder Reste von geprüften Erzeugnissen auf der Berührungsfläche zwischen dem Boden des Prüfraumes und der Schutzhaube die Abdichtung des Prüfraumes gegenüber der Umgebung nicht nachteilig beeinflussen können.

Eine weitere vorteilhafte Ausgestaltung der Erfindung sieht vor, dass der Aufnahmeraum nur einen Teil der Fläche unter dem Boden des Prüfraumes einnimmt, so dass ein zusätzlicher, durch eine Schwenktür oder dergleichen von der Umgebung abgetrennter und vom Aufnahmeraum wasser- und staubdicht abgetrennter Raum entsteht, in den die von außen zugängliche Schnittstelle des Aufnahmeraumes mündet. Die geprüften Erzeugnisse können durch eine Auswurföffnung im Boden des Prüfraumes in einem unter dem im Boden des Prüfraums angeordneten Sammelbehälter aufgefangen werden.

Eine vorteilhafte Ausgestaltung der Erfindung sieht vor, dass die Transporteinrichtung ein um seine vertikale Mittelachse rotierend angetriebener Transportstern ist, der an seinem Außenumfang mindestens eine in Richtung des Prüfraumbodens offene Aufnahmezelle für ein zu prüfendes Erzeugnis aufweist, und der durch seine Rotation die in den Zellen liegenden Erzeugnisse verschiedenen Prüfeinrichtungen zuführt, wobei die Prüfungen an dem in der Aufnahmezelle auf dem Prüfraumboden liegenden Erzeugnis durchgeführt werden, so dass durch die Weiterdrehung des Transportsterns das Erzeugnis einer anderen Prüfeinrichtung zugeführt werden kann und schließlich beim Überfahren der Auswurföffnung in diese hineinfällt.

Eine andere vorteilhafte Ausgestaltung der Erfindung sieht vor, dass die Auswurföffnung in den vom Aufnahmeraum wasser- und staubdicht abgetrennten und gegenüber der Umgebung durch eine Schwenktür oder dergleichen verschließbaren Raum mündet, von dem aus auch die von außen zugängliche Schnittstelle des Aufnahmeraumes erreichbar ist.

Eine besonders vorteilhafte Ausgestaltung der Erfindung sieht vor, dass, um insbesondere eine spritzwasserdichte Anordnung einer Waage im Prüfraum zu erreichen, bei dieser der Waagentisch erhöht über dem Boden des Prüfraumes angeordnet ist. Der Boden des Prüfraumes weist unterhalb des Waagentischs einen Süllrand auf, welcher in Verbindung mit dem eine Ausnehmung zur Aufnahme des Süllrands aufweisenden Waagentisch eine flüssigkeits- und staubdichte Labyrinthdichtung bildet, so dass keine Reinigungslösung in das darunter liegende Gehäuse eindringen kann. Der Süllrand kann dabei gleichzeitig als Wegbegrenzung für den Waagentisch dienen, so dass eine Überlastung der empfindlichen Waage beim Reinigen ausgeschlossen werden kann. Die Anordnung erlaubt darüber hinaus den Wagentisch nach oben hin abzunehmen, um Wartungsarbeiten oder dergleichen durchzuführen, beispielsweise um die Führung des Stempels, mit dem der Waagentisch auf die Kraftmessdose drückt, zu reinigen. Die Abdichtung der Antriebswelle des Transportsterns kann ebenfalls mittels eines Sülls erfolgen, vorteilhafterweise wird jedoch entweder anstatt des Süllrandes oder zusätzlich zu diesem ein axial abdichtendes Lager für die Durchführung der Antriebswelle durch den Prüfraumboden verwendet.

Eine andere besonders vorteilhafte Ausgestaltung der Erfindung sieht vor, dass um die Zufuhr der Prüfgegenstände auf den über dem Prüfraumboden angeordneten Waagentisch mittels eines Transportsterns zu ermöglichen, zwischen Prüfraumboden und Transportstern mindestens eine ein- oder mehrteilige Platte auf den Prüfraumboden aufgelegt wird, welche Öffnungen für die Antriebswelle des Transportsterns, den Waagentisch, die Auswurföffnung für die Prüfgegenstände und gegebenenfalls für andere Prüfeinrichtungen und für den Antrieb der Ladeeinrichtung aufweist, und die in ihrer Dicke der Bauhöhe des Waagentisches über dem Prüfraumboden entspricht. Sowohl der Transportstern als auch die darunter angeordnete Platte sind nach oben hin entfernbar angeordnet um beispielsweise nach deren Entfernen Reinigungsarbeiten einfach ausführen zu können.

Eine vorteilhafte Ausgestaltung der Erfindung sieht vor, die Kraftmessdose der Waage direkt unter dem Waagentisch anzuordnen, um auf die Abdichtung der Durchführung des auf die Kraftmessdose drückenden Stempels des Waagentisches durch die Gehäusewandung verzichten zu können und nur die elektrischen und ggf. optischen Verbindungen der Kraftmessdose mit den Signalverstärkern im inneren des Gehäuses durch die Gehäusewandung führen zu müssen.

Eine vorteilhafte Ausgestaltung der Erfindung sieht vor, die Auswurföffnung mit einem Süllrand zu versehen, um ein Abfließen der Reinigungslösung durch die Auswurföffnung zu verhindern, wobei die Höhe des Süllrandes über dem Boden des Prüfraumes geringer ist, als der Abstand der Unterseite des Transportsterns vom Boden des Prüfraumes.

Eine andere vorteilhafte Ausgestaltung der Erfindung sieht vor, die Auswurföffnung während der Reinigungsarbeiten mit einem Pfropfen oder dergleichen zu verschließen bzw. verschließbar auszugestalten.

In weiterer Ausgestaltung der Vorrichtung ist in dem durch die Schwenktür zugänglichen, vom Aufnahmeraum abgetrennten Raum unter dem Boden des Prüfraumes zusätzlich zu dem Sammelbehälter ein Computer oder dergleichen für die Messwerteaufzeichnung und -weiterverarbeitung angeordnet, wobei der Computer mittels in der Trennwand zum Aufnahmeraum angeordneten, elektrischen und/oder optischen Anschlüssen mit der Signalaufbereitung und Signalverstärkung der Waage, den Steuerungen für die Antriebe von Vereinzelungsrinne, Ladeeinrichtung und Transportstern, sowie gegebenenfalls zusätzlichen, mit der Wiegeeinrichtung verbundenen Mess- oder Prüfeinrichtungen verbindbar ist.

Eine zusätzliche vorteilhafte Ausgestaltung der Erfindung sieht vor, dass die Schutzhaube mittels einer Verriegelung, die nur von dem durch die Schwenktür zugänglichen, vom Aufnahmeraum abgetrennten Raum unter dem Boden des Prüfraumes aus bedient werden kann, abschließen zu können.

Eine zusätzliche vorteilhafte Ausgestaltung der Erfindung sieht vor, einen steten Überdruck im Aufnahmeraum unter Prüfraumboden zu erzeugen, so dass ein konstanter Luftstrom insbesondere während des Reinigens durch eventuell vorhandene Spalte an den Durchführungen der Antriebswellen und dergleichen am Gehäuse austritt.

Eine weitere vorteilhafte Ausgestaltung der Erfindung sieht vor, den Prüfraum mit einem konstanten Unterdruck zu beaufschlagen um so bei der Prüfung beispielsweise von Hormonprodukten oder Antibiotika ein Austreten von Abriebstaub oder dergleichen aus dem Prüfraum zu verhindern.

In einer weiteren vorteilhaften Ausgestaltung der Vorrichtung ist das gegenüber dem Prüfraum hermetisch abgekapselte Gehäuse mit dem von diesem umschlossenen Aufnahmeraum statt unter dem Boden des Prüfraumes neben demselben angeordnet.

Gemäß einer weiteren Variante der Erfindung besteht die Vorrichtung aus einem Gehäuse mit einem darüber angeordneten, verschließbaren Prüfraum, der durch einen Boden und eine darauf aufsetzbare, bewegliche Schutzhaube gebildet ist, in welchem Prüfraum sich die Prüfeinrichtung sowie die Transporteinrichtung befinden, wobei Antriebe und gegebenenfalls Versorgungseinrichtungen für die im Prüfraum angeordneten Prüf- und Transporteinrichtung in dem gegenüber dem Prüfraum - gegebenenfalls auch gegenüber der übrigen Umgebung - wasserdicht und staubdicht gekapselten separaten Gehäuse angeordnet sind.

Gemäß einer weiteren Variante der Erfindung sind wenigstens Teile der Zuleitungen zu den Antrieben und den Versorgungseinrichtungen für die Energieversorgung der Prüfeinrichtung sowie der Transporteinrichtung innerhalb des Gehäuses in wasserdicht und staubdicht gekapselter Weise nach außerhalb des Prüfraumes geführt.

Gemäß einer weiteren Variante der Erfindung sind die Durchbrüche durch den Boden zur Durchführung der Zuleitungen sowie zur Aufnahme von Prüfeinrichtungen mit wasserdichten Dichtungen ausgestattet.

Gemäß einer weiteren Variante der Erfindung weist die Vorrichtung eine innerhalb des Prüfraumes als Transporteinrichtung angeordnete Vereinzelungseinrichtung auf, welcher die pharmazeutischen Erzeugnisse aus einem Speicherbehälter zuführbar sind, wobei die Vorrichtung eine über dem Boden des Prüfraumes angeordneten weitere Transporteinrichtung aufweist, welche mindestens eine zum Boden hin offenen Aufnahmezelle aufweist, in welche jeweils ein Erzeugnis aus der Vereinzelungseinrichtung zuführbar ist, wobei das Erzeugnis in der Aufnahmezelle mindestens einer vorzugsweise unter der Transporteinrichtung angeordneten Prüfeinrichtung zur Prüfung des Erzeugnisses auf Gewicht und/ oder Abmessungen und/oder Härte und/oder Abriebeigenschaften zuführbar ist, und mittels einer nach der Prüfeinrichtung im Boden angeordneten Auswurföffnung, in welche das Erzeugnis nach Prüfung und Fortsetzung des Transportweges hineinfällt, aus der Aufnahmezelle entfernbar ist, wobei unterhalb der Auswurföffnung ein Sammelbehälter zur Aufnahme der geprüften Erzeugnisse oder deren Teile angeordnet ist.

Gemäß einer weiteren Variante der Erfindung ist die Prüfeinrichtung eine Waage mit einem Waagentisch und einer Kraftmessdose, wobei der Waagentisch über dem Boden des Prüfraums angeordnet ist und der Boden unter dem Waagentisch oder innerhalb des Waagentisches eine wasserdichte Dichtung aufweist.

Gemäß einer weiteren Variante der Erfindung weist der Boden unter dem Waagentisch einen Süllrand auf, wobei der Waagentisch eine Ausnehmung zur Aufnahme des Süllrandes besitzt, so dass durch das Zusammenwirken von Süllrand und Ausnehmung am Waagentisch eine wasserdichte Dichtung, insbesondere Labyrinthdichtung, entsteht.

Gemäß einer weiteren Variante der Erfindung sind die Antriebe und gegebenenfalls Versorgungseinrichtungen der im Prüfraum angeordneten Prüfeinrichtungen und Transporteinrichtungen in dem gegenüber dem Prüfraum und der Umgebung mindestens spritzwasser- und staubdicht gekapselten separaten Gehäuse angeordnet, wobei wenigstens Teile der Zuleitungen zu den Antrieben und den Versorgungseinrichtungen für die Energieversorgung der Prüfeinrichtung innerhalb des Gehäuses in ebenfalls mindestens spritzwasser- und staubdicht gekapselter Weise nach außerhalb des Prüfraumes geführt sind.

Gemäß einer weiteren Variante der Erfindung ist das gegenüber dem Prüfraum hermetisch abgekapselte Gehäuse mit dem von diesem umschlossenen Aufnahmeraum statt unter dem Boden des Prüfraumes neben demselben angeordnet.

Kurzbezeichnung der Zeichnung, in der zeigen:
- Figur 1: eine Vorderansicht einer erfindungsgemäßen Vorrichtung
- Figur 2: eine um 90 Grad nach rechts geklappte Seitenansicht der Vorrichtung der Figur 1
- Figur 3: eine Draufsicht auf die Vorrichtung der Figur 1,
- Figur 4: eine Draufsicht auf die Vorrichtung der Figur 1 bei entfernter Schutzhaube,
- Figur 5: eine perspektivische Ansicht der Vorrichtung von Figur 1 mit Vereinzelungseinrichtung, Ladeeinrichtung, Transportstern und geschlossener Schutzhaube sowie geschlossener Schwenktür,
- Figur 6: eine perspektivische Ansicht der Vorrichtung von Figur 1 mit geöffneter Schutzhaube, wobei die Schwenktür nicht dargestellt ist,
- Figur 7: eine perspektivische Vorderansicht der Vorrichtung von Figur 1 bei abgenommener Frontwand, wobei die Schutzhaube nicht und die Schwenktür nur teilweise dargestellt ist, und
- Figur 8: eine perspektivische Draufsicht der Vorrichtung von Figur 1 bei geöffneter Schutzhaube, wobei die Vereinzelungseinrichtung und der Transportstern nicht und die Schutzhaube und die Ladeeinrichtung jeweils nur teilweise dargestellt sind.

Die Figuren sind lediglich schematische Darstellungen.

### Beschreibung des Ausführungsbeispiels:

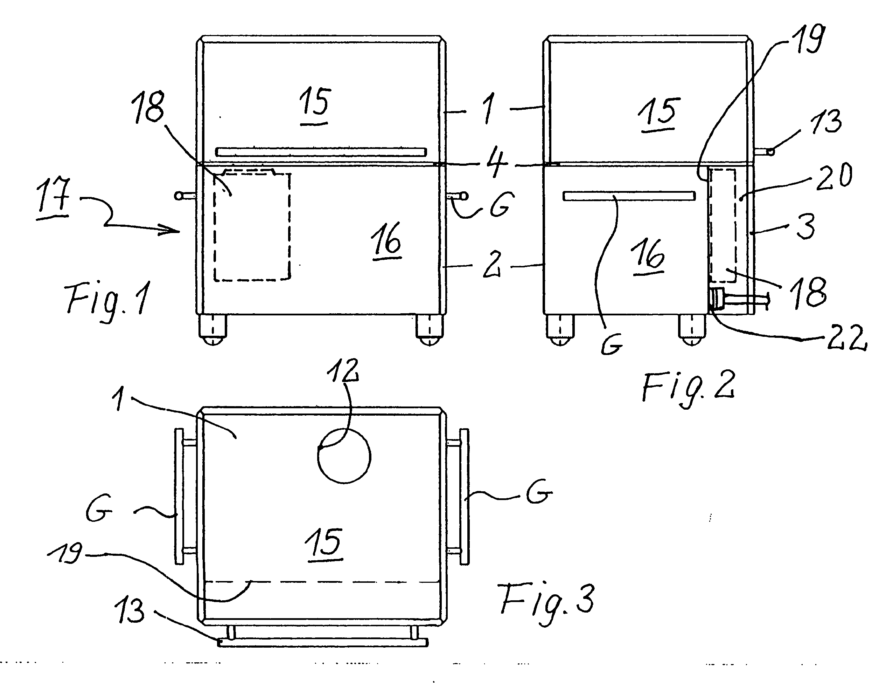

Die in den Figuren 1, 2 und 3 in einer Vorderansicht, einer um 90 Grad nach rechts geklappten Seitenansicht und in einer Draufsicht dargestellte Vorrichtung 17 zur Qualitätskontrolle fester, pharmazeutischer Erzeugnisse besteht aus einem quaderförmigen Gehäuse 2 und weist einen durch eine transparente Schutzhaube 1 geschlossenen Prüfraum 15 auf, wobei die Schutzhaube 1 an ihrer Vorderseite einen Griff 13 zum Anheben und Schwenken der Schutzhaube 1 aufweist. Ebenso weist die Schutzhaube 1 in ihrer oberen Deckfläche eine Öffnung 12 auf, durch welche die pharmazeutischen Erzeugnisse in den Prüfraum 15 eintreten.

Der Prüfraum 15 ist durch einen Prüfraumboden 4 vom Gehäuse 2 abgetrennt, so dass unterhalb des Prüfraumbodens 4 der Vorrichtung 17 innerhalb des Gehäuses 2 ein wasser- und staubdichter Aufnahmeraum 16 zur Unterbringung von notwendigen Steuerungen und Antrieben der im Prüfraum 15 angeordneten Einrichtungen für Vereinzelung, Transport und Prüfung von Pillen, Tabletten oder dergleichen, angeordnet ist. Ebenso sind im Aufnahmeraum 16 Signalverstärker der Prüfeinrichtungen für beispielsweise Gewicht, Abmessungen und Härte angeordnet, welche wasserdicht gekapselt sind.

Eine Schwenktür 3, welche an der Vorderseite des Gehäuses 2 der Vorrichtung 17 angebracht ist, verschließt den innerhalb des Gehäuses 2 wasser- und staubdicht abgetrennten Aufnahmeraum 16 unter dem Prüfraumboden 4 der Vorrichtung 17, in welchem Aufnahmeraum 16 beispielsweise ein Computer oder dergleichen für die Prüfdatenerfassung untergebracht werden kann. Die vordere Wandung 19 des Gehäuses 2 ist gegenüber dem Prüfraumboden 4 zurückgesetzt, wie es der Figur 2 zu entnehmen ist, so dass zwischen der Schwenktür 3 und der Vorderwandung 19 des Gehäuses 2 ein durch die Schwenktür 3 abgedeckter Freiraum 20 ausgebildet ist, innerhalb desselben ein Sammelbehälter 18 für die geprüften Erzeugnisse angeordnet ist. Um auch den Sammelbehälter 18 abzudecken und zur Ausbildung des Freiraums 20 weist die Schwenktür 3 an ihren beiden Enden Winkel auf, so dass die Schwenktür 3 in der Draufsicht doppel-L-förmig gestaltet ist. Innerhalb der Vorderwandung 19 des Gehäuses 2 ist auch eine nicht gezeigte Schnittstelle zum Anschluss sämtlicher elektrischen, mechanischen oder pneumatischen Aggregate angeordnet. Die Vorrichtung, mit Ausnahme der Schutzhaube 1, besteht vorzugsweise aus rostfreiem Edelstahl.

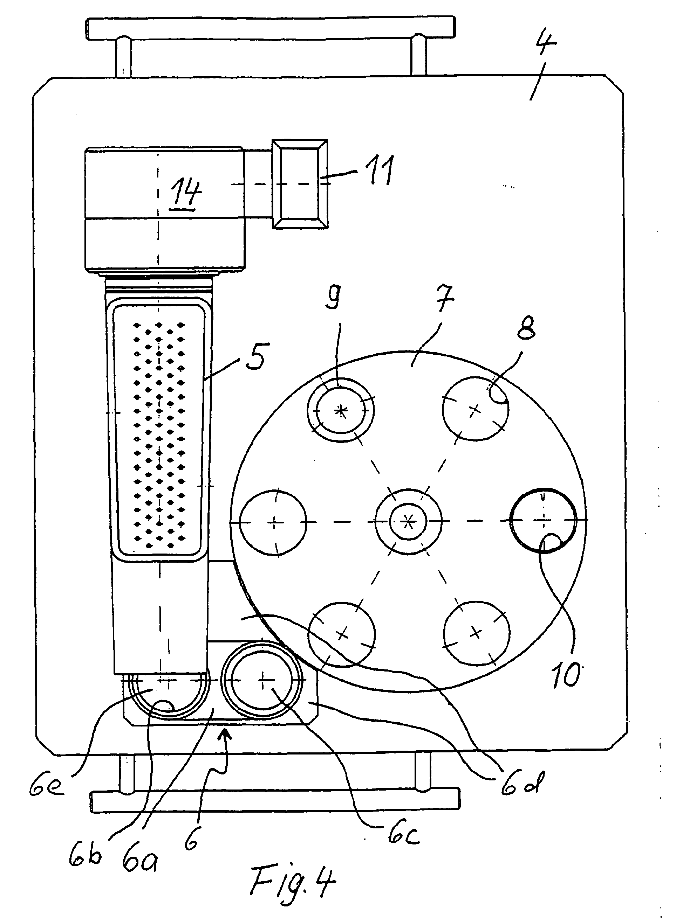

Figur 4 zeigt eine Draufsicht auf eine erfindungsgemäße Vorrichtung zur Qualitätskontrolle fester, pharmazeutischer Erzeugnisse bei abgenommener Schutzhaube 1. Von einem nicht dargestellten Speicherbehälter, der innerhalb oder außerhalb des Prüfraumes 15 angeordnet sein kann, werden die zu prüfenden Erzeugnisse, beispielsweise Tabletten, einer Vereinzelungseinrichtung 5 zugeführt, welche im gezeigten Beispiel eine Schüttelrinne 5 ist, welche mit einem Elektromotor 14 angetrieben wird. Die Erzeugnisse fallen am Ende der Vereinzelungseinrichtung 5 einzeln in eine Ladeeinrichtung 6. Die Ladeeinrichtung 6 ist um eine Achse schwenkbar angeordnet und führt die Tabletten einzeln einem drehbar angeordneten Transportstern 7 zu, der über mehrere, über seinem Umfang peripher verteilte Aufnahmekammern 8 verfügt. Zwischen dem zum Betrachter hin nach oben abnehmbaren Transportstern 7 und dem Prüfraumboden 4 ist eine ebenfalls zum Betrachter hin abnehmbare Platte angeordnet, welche Ausnehmungen für die Antriebswelle für die Schwenkbewegung der Ladeeinrichtung 6, für die Antriebswelle des Transportsterns 7, die Waage 9 und für die Auswurföffnung 10 aufweist. Im Bereich um die Ladeeinrichtung 6 ist die Platte um die Dicke des Transportsterns 7 erhöht, so dass die Ladeeinrichtung 6 bündig über dem Transportstern 7 angeordnet ist und die Erzeugnisse von der Ladeeinrichtung 6 so über den Rand des Transportsterns 7 geschoben werden können, dass sie in die Aufnahmekammern 8 des Transportsterns 7 von oben hineinfallen. Die Aufnahmekammern 8 sind Durchgangslöcher und somit in Richtung Prüfraumboden 4 offen.

Durch die Drehbewegung des Transportsterns 7 um seine senkrecht zur Betrachtungsebene stehende Mittelachse im Uhrzeigersinn werden die Tabletten über einen gegenüber dem Prüfraumboden 4 um die Dicke der zwischen Transportstern 7 und Prüfraumboden 4 angeordneten Platte erhöht angeordneten Waagentisch 9 einer im Gehäuse 2 angeordneten Waage geführt, welche bündig mit der Platte abschließt. Danach werden die gewogenen Erzeugnisse durch die Drehbewegung des Transportsterns 7 über eine Auswurföffnung 10 geführt, durch die sie in den darunter, hinter der Schwenktür 3 angeordneten Sammelbehälter 18 fallen.

Um den Aufnahmeraum 2 wasserdicht auszuführen, ist beispielsweise der Elektromotor 14 der Vereinzelungseinrichtung 5 durch ein auf dem Boden 4 des Prüfraumes aufgeschweißtes und in Richtung des Betrachters verschlossenes Vierkantrohr 11 geführt, so dass nur Durchführungen für Antriebswellen und für den auf die Kraftmessdose der Waage drückenden, unter dem Waagentisch 9 angeordneten Stempel durch das ansonsten wasser- und staubdicht verschließbaren Aufnahmeraum 3 geführt sind.

Zum Reinigen werden verfahrensmäßig sämtliche Zuführ- und Abführöffnungen oder Zulauf- und Ablauföffnungen diese hermetisch wasserdicht verschlossen bis auf eine Öffnung zum Entweichen der Luft. Dann wird in den Prüfraum 15 Wasser eingefüllt, beispielsweise durch eine hierfür vorgesehenen Schlauchanschluss, und auch die Öffnung für das Entweichen der Luft aus dem Prüfraum 15 verschlossen. Nunmehr werden sämtliche beweglichen Aggregate, wie Schüttelrinne 5, Transportsterns 7, Ladeeinrichtung 6, u.U. auch die Antriebswelle für die Schwenkbewegung der Ladeeinrichtung 6, durch ihre Antriebsaggregate innerhalb des Wasserbades in Bewegung versetzt, so dass durch die Bewegung der Aggregate innerhalb des Wasserbades eine große Turbulenz entsteht und dadurch eine gründliche Reinigung stattfindet. Nach dem Ablassen des Wassers kann auch eine Lufttrocknung mittels heißer Luft erfolgen, welche gleichermaßen durch die Zuführöffnung zugeführt und durch die Abführöffnung abgeblasen wird.

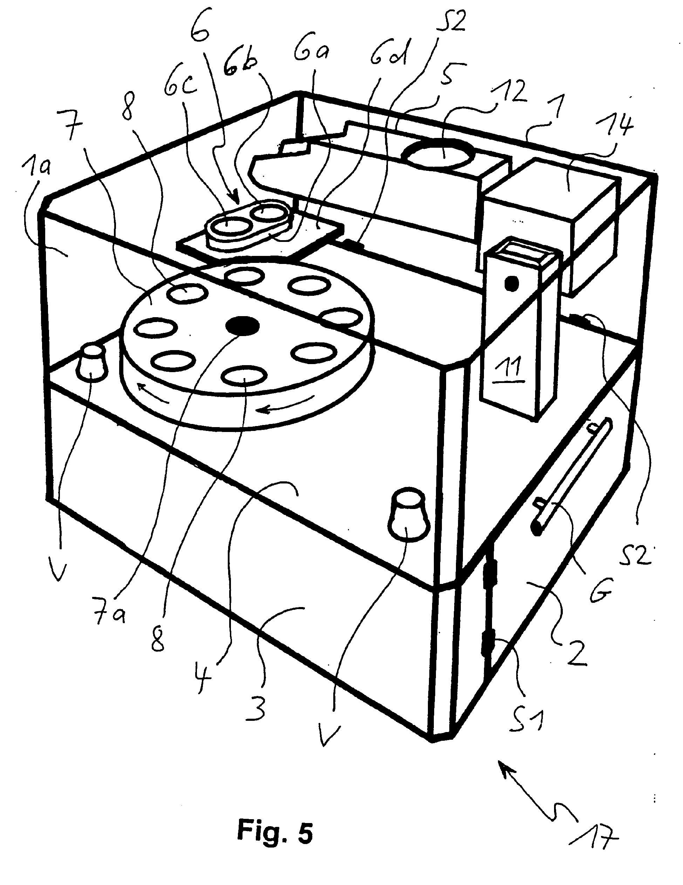

Figur 5 zeigt eine schematische perspektivische Ansicht der Vorrichtung 17 von Figur 1 mit der Vereinzelungseinrichtung 5, der Ladeeinrichtung 6, dem Transportstern 7 und geschlossener Schutzhaube 1 sowie geschlossener Schwenktür 3. Die Schwenktür 3 ist mittels Scharnieren S1 am Gehäuse 2 angelenkt.

Die Vorrichtung 17 dient erfindungsgemäß zur Qualitätskontrolle fester pharmazeutischer Erzeugnisse, wie Tabletten, Pillen, Oblongs oder dergleichen. Das Gehäuse 2 ist unter dem gegenüber der Umgebung spritzwasserdicht oder wasser- und staubdicht verschließbaren Prüfraum 15 angeordnet, welcher durch den Boden 4 und die darauf aufsetzbare und abnehmbare Schutzhaube 1 gebildet ist.

Der Boden 4 des Prüfraumes 15 bildet zugleich die obere Wandung des Gehäuses 2 und des zwischen der Schwenktür 3 und dem Gehäuse befindlichen und daher in Fig. 5 nicht einsehbaren Vorraumes 20. Der Griff 13 zum Öffnen der Schutzhaube 1 ist in Fig. 5 nicht dargestellt.

Die Schutzhaube 1 ist mittels Scharnieren S2 am Gehäuse angelenkt und mittels Verriegelungen V wasser- und staubdicht auf den Boden 4 aufgepresst. Die Verriegelungen V weisen zu diesem Zweck je einen Fortsatz auf, welcher auf einen auf der Innenseite der Vorderwand 1a der Schutzhaube 1 angeordneten Vorsprung eine Anpresskraft in Richtung des Bodens 4 auszuüben imstande ist. Diese Fortsätze und Vorsprünge sind in den Figuren nicht dargestellt. Die Verriegelungen V lassen sich mit Hilfe von Bedienelementen, welche ebenfalls nicht gezeigt sind, aktivieren und deaktivieren, d.h. die Schutzhaube lässt sich mittels der Bedienelemente verriegeln und entriegeln. Das Gehäuse 2 trägt aus Gründen der einfacheren Handhabbarkeit seitliche Griffe G.

Zur Prüfung pharmazeutischer Erzeugnisse werden dieselben durch die Einfüllöffnung hindurch in die Vereinzelungseinrichtung 5 eingebracht. Die Wirkungsweise einer derartigen Vereinzelungseinrichtung ist in dem Patent DE 38 64 82.2 beschrieben.

Der Elektromotor 14 ist mittels des Vierkantrohres 11 aufgeständert.

Die Ladeeinrichtung 6 weist einen Umfassungskörper 6a mit einer Aussparung 6b, eine Ladewelle 6c und eine plattenförmige Unterlage 6d auf. Der Umfassungskörper 6a ist an der Ladewelle 6c drehfest angeordnet. Die Vereinzelungseinrichtung 5 an dem Elektromotor 14 so angeordnet, dass die vereinzelten Erzeugnisse aus der Vereinzelungseinrichtung 5 einzeln nacheinander in eine oben und unten offene Aussparung 6b des Umfassungskörpers 6a fallen.

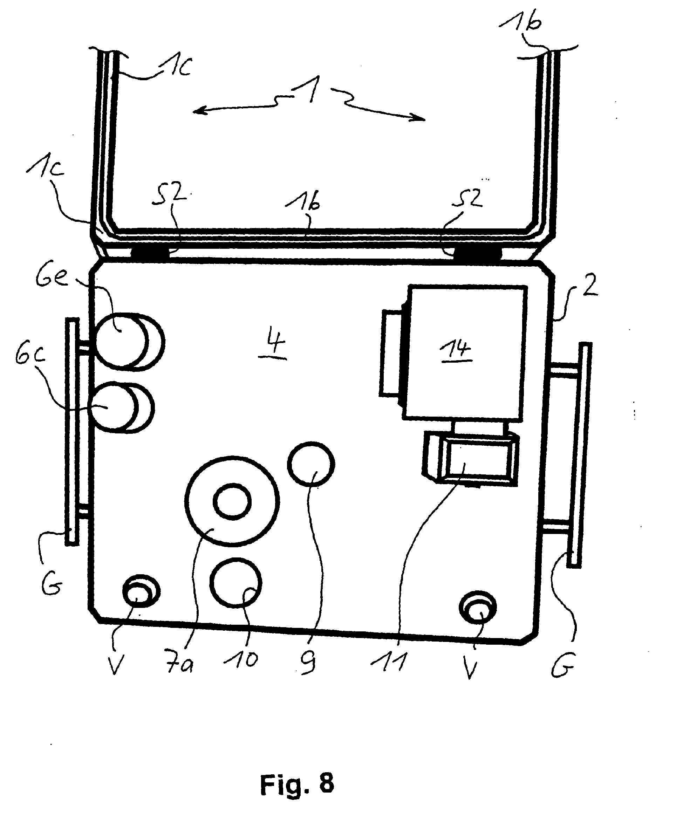

Unterhalb der Aussparung 6a befindet sich ein in Fig. 5 nicht dargestellter Aufprallsensor 6e (vgl. Fig. 8), welcher den Einfall eines Erzeugnisses in die Aussparung 6b detektiert und mit Hilfe eines weiteren (nicht gezeigten) Elektromotors eine Ladewelle 6c in eine solche Schwenkbewegung versetzt, dass sich die Aussparung 6b über eine der oben und unten offenen Aufnahmezellen 8 des Transportsterns 7 bewegt. Der Transportstern 7 ist mittels einer Rotationswelle 7a schrittweise rotierbar. Die Rotationsbewegung des Transportsterns 7 ist hierbei mit der Schwenkbewegung so synchronisiert, dass sich die Aussparung 6b am Ende jeder Schwenkbewegung über einer der Aufnahmezellen 8 befindet. Das Erzeugnis wird also durch die Schwenkbewegung vom Aufprallsensor 6a über die Unterlage 6d zu einer Aufnahmezelle 8 transportiert und fällt in diese hinein. Dieser Vorgang wiederholt sich jedes Mal, wenn eines der zu prüfenden Erzeugnisse in die Aussparung 6b fällt. Die Oberseiten des Aufprallsensors 6e, der Unterlage 6d und des Transportsterns 7 befinden sich bevorzugt auf gleicher Höhe.

Die Prüfung des pharmazeutischen Erzeugnisses besteht im vorliegenden Beispiel auf einer Wägung. Der Transportstern 7 dreht sich, nachdem das Erzeugnis in die Aufnahmezelle gefallen ist, und bewegt das Erzeugnis auf diese Weise auf die Waage 9, welche in Fig. 5 nicht gezeigt ist, und nach erfolgter Wägung zu der unter dem Transportstern 7 angeordneten Auswurföffnung 10 (in Fig. 5 nicht gezeigt), so dass das Erzeugnis in diese hineinfällt und in den Sammelbehälter 18 gelangt.

Die Vereinzelungseinrichtung 5, die Ladeeinrichtung 6 und der Transportstern 7 bilden zusammen eine Transporteinrichtung für die Erzeugnisse. Selbstverständlich können weitere Parameter des einzelnen pharmazeutischen Erzeugnisses geprüft bzw. gemessen werden, z.B. die Höhe und/oder die Berstkraft des Erzeugnisses, bevor dieses der Auswurföffnung 10 zugeführt wird.

Erfindungsgemäß ist der Prüfraum 15 spritzwasserdicht oder wasser- und staubdicht verschließbar. Dies ist insbesondere dann äußerst vorteilhaft, wenn der Prüfraum 15, nachdem umweltschädliche oder gesundheitsgefährdende pharmazeutische Erzeugnisse mit der Vorrichtung 17 geprüft wurden, gereinigt wird, z.B. durch Spülung, da auf diese Weise gewährleistet ist, dass keine Reste oder Abrieb der Erzeugnisse unkontrolliert in die Umwelt gelangen. Im Fall einer wasserdichten Ausführung kann hierbei vorteilhafterweise sogar eine vollständige Flutung des Prüfraumes 15 vorgenommen werden.

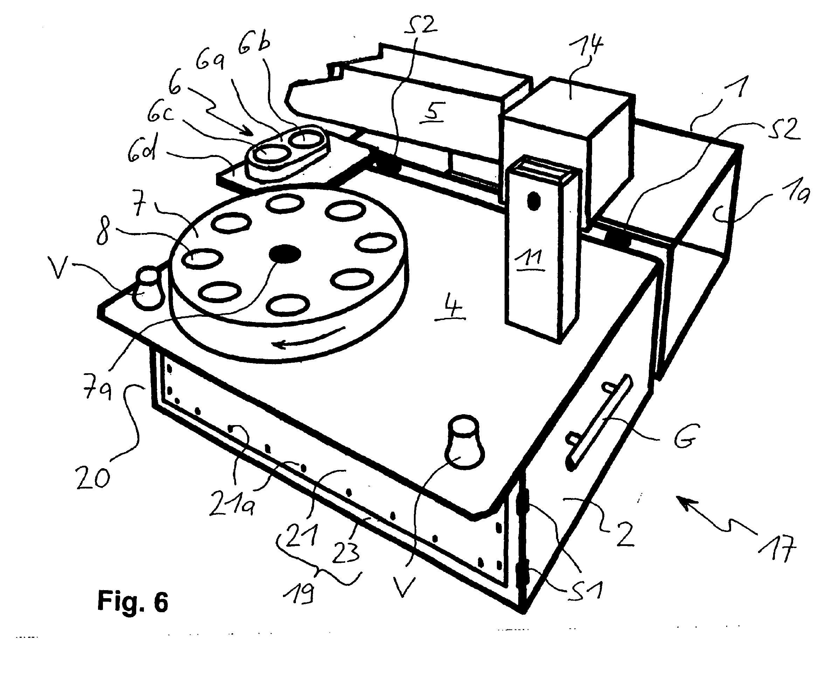

Figur 6 zeigt eine perspektivische Ansicht der Vorrichtung 17 mit vollständig geöffneter Schutzhaube 15, wobei die Schwenktür 3 von Fig. 5 nicht dargestellt ist. Der Vorraum 20 ist daher in Fig. 6 frei einsehbar. Die Vorderwandung 19 des Gehäuses 2 besteht im Beispiel von Fig. 6 aus einem umlaufenden Rahmen 23 und einer darauf aufgesetzten und mit Schrauben 21a befestigten Frontplatte 21. Gemäß einer bevorzugten Ausgestaltung der Erfindung ist auch das Gehäuse 2 spritzwasserdicht oder wasser- und staubdicht verschließbar ausgeführt. Vorzugsweise ist die Frontplatte 21 daher spritzwasserdicht oder wasser- und staubdicht auf den Rahmen 23 aufgesetzt. Die Schutzhaube 1 ist in Fig. 6 um die Scharniere S2 nach hinten geschwenkt.

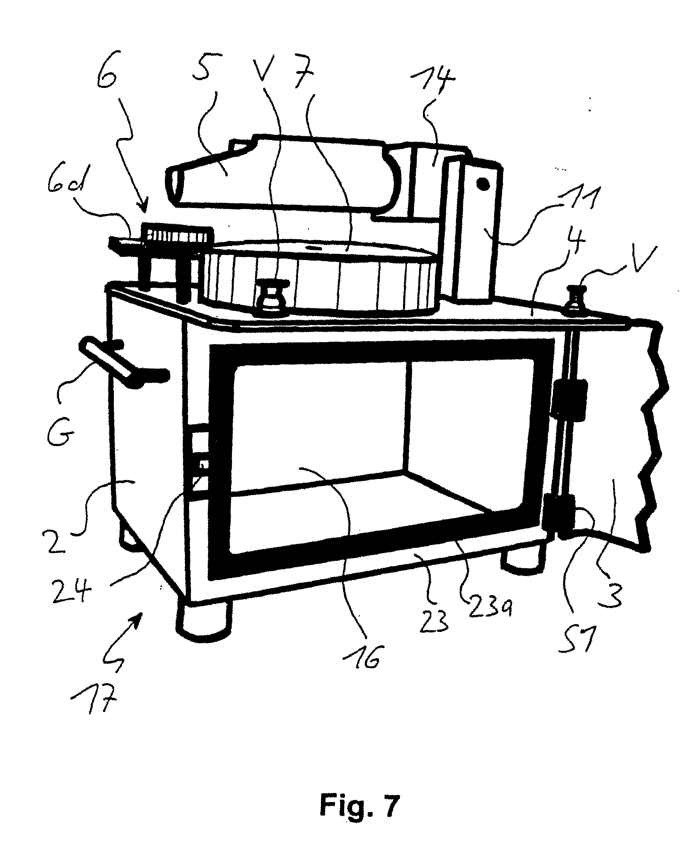

Figur 7 zeigt eine perspektivische Vorderansicht der Vorrichtung 17 bei abgenommener Frontwand 21, wobei die Schutzhaube 1 nicht und die geöffnete Schwenktür 3 nur teilweise dargestellt ist. Auf dem Rahmen 23 liegt entlang seiner Innenkanten eine umlaufende Dichtung 23a auf, welche zur Abdichtung der Frontplatte 21 gegenüber dem Rahmen 23 dient. Am Rahmen 23 ist ferner gegenüber den Scharnieren S1 ein Schließmechanismus 24 angeordnet, welcher zum Verriegeln der Schwenktür 3 in geschlossener Stellung dient.

Figur 8 zeigt eine perspektivische Draufsicht der Vorrichtung 17 bei geöffneter Schutzhaube, wobei die Vereinzelungseinrichtung 5 und der Transportstern 7 nicht dargestellt sind und die Schutzhaube 1 sowie die Ladeeinrichtung 6 jeweils nur teilweise dargestellt sind. Die Schutzhaube 1 ist vollständig geöffnet und befindet sich in der bereits in Fig. 6 gezeigten Stellung. Diejenige Stirnfläche 1c der Schutzhaube 1, welche in geschlossener Stellung der Schutzhaube 1 nach unten weist und somit dem Boden 4 zugewandt ist, weist eine umlaufende Dichtung 1b auf, welche imstande ist, die Stirnfläche 1c spritzwasserdicht oder wasser- und staubdicht gegen den Boden 4 abzudichten.

Der Transportstern 7 ist in Fig. 8 nicht gezeigt, so dass der Blick auf die Rotationswelle 7a sowie die Waage 9 und die Auswurföffnung 10 freigegeben ist. Ebenso sind der Umfassungskörper 6a und die Unterlage 66d der Ladeeinrichtung in Fig. 8 nicht dargestellt, so dass der über den Boden 4 nach oben hinausragende Teil der Schwenkwelle 6c und der Aufprallsensor 6e vollständig sichtbar sind.

### Gewerbliche Anwendbarkeit:

Der Gegenstand der Erfindung ist insbesondere in der pharmazeutischen Industrie bei der Überprüfung und Bearbeitung, Handling, von pharmazeutischen Erzeugnissen, wie Tabletten, anwendbar. Die besondere Nützlichkeit besteht darin, dass die Vorrichtung absolut hygienisch zu arbeiten imstande ist und insbesondere ein Wechsel von unterschiedlichen Medikamenten oder Pillen innerhalb der Vorrichtung keinen Einfluss auf die saubere Verarbeitung des nachfolgen zu verarbeitenden Gutes hat, weil vor dem Wechsel des zu verarbeitenden Gutes die Vorrichtung optimal gereinigt werden kann.

### Bezugszahlenliste:

- 1: Schutzhaube
- 1a: Vorderseite von 1
- 1b: Dichtung von 1
- 1c: untere Stirnfläche von 1
- 2: Gehäuse
- 3: Schwenktür
- 4: Boden des Prüfraumes
- 5: Vereinzelungseinrichtung
- 6: Ladeeinrichtung
- 6a: Umfassungskörper von 6
- 6b: Aussparung von 6
- 6c: Schwenkwelle von 6
- 6d: Unterlage von 6
- 6e: Aufprallsensor von 6
- 7: Transportstern
- 7a: Rotationswelle von 7
- 8: Aufnahmezelle
- 9: Waage
- 10: Auswurföffnung
- 11: Vierkantrohr
- 12: Einfüllöffnung
- 13: Griff zum Öffnen der Schutzhaube
- 14: Elektromotor
- 15: Prüfraum unter der Schutzhaube 1
- 16: Aufnahmeraum des Gehäuses 2
- 17: Vorrichtung
- 18: Sammelbehälter
- 19: Vorderwandung des Gehäuses 2
- 20: Vorraum
- 21: Frontplatte
- 21 a: Schrauben
- 22: Schnittstelle
- 23: Rahmen
- 24: Schließmechanismus
- G: Griff
- S1, S2: Scharniere
- V: Verriegelungen

## Patentansprüche

1. Vorrichtung (17) zur Qualitätskontrolle fester pharmazeutischer Erzeugnisse, wie Tabletten, Pillen, Oblongs oder dergleichen, mit wenigstens einer Prüfeinrichtung (9), welche mindestens einem Sensor zur Prüfung und Erfassung eines oder mehrerer Parameter des einzelnen pharmazeutischen Erzeugnisses aufweist, wie Waage (9) und/oder Höhenmesseinrichtung und/oder Kraftmesseinrichtung zur Messung der Berstkraft des Erzeugnisses, sowie mit einer Transporteinrichtung (5,6,7) für die Erzeugnisse, **dadurch gekennzeichnet,**
**dass** die Vorrichtung (17) ein Gehäuse (2) und einen darüber angeordneten, gegenüber der Umgebung spritzwasserdicht oder wasser- und staubdicht verschließbaren Prüfraum (15) aufweist, welcher durch einen Boden (4) und eine darauf aufsetzbare und abnehmbare Schutzhaube (1) gebildet ist und in welchem sich die Prüfeinrichtung (9) sowie die Transporteinrichtung (5,6,7) befinden.

2. Vorrichtung nach Anspruch 1, **dadurch gekennzeichnet,**
**dass** die Schutzhaube (1) in lösbarer Weise spritzwasserdicht oder wasser- und staubdicht mit dem Boden (4) des Prüfraumes (15) verbindbar ist.

3. Vorrichtung nach Anspruch 1, **dadurch gekennzeichnet,**
**dass** die Schutzhaube (1) spritzwasserdicht oder wasser- und staubdicht peripher auf den Boden (4) des Prüfraumes (15) aufpressbar und verriegelbar aufgesetzt ist, wobei die Schutzhaube (1) in ihrer dem Boden (4) des Prüfraums (15) zugewandten Stirnfläche (1c) eine umlaufende spritzwasserdichte oder wasser- und staubdichte Dichtung (1 b) aufweist

4. Vorrichtung nach einem der Ansprüche 1 bis 3, **dadurch gekennzeichnet,**
**dass** das Gehäuse (2) ebenfalls spritzwasserdicht oder wasser- und staubdicht verschließbar ist.

5. Vorrichtung nach einem der Ansprüche 1 bis 4, **dadurch gekennzeichnet,**
**dass** in dem Gehäuse (2) Einrichtungen zum Antrieb, zur Steuerung und zur Versorgung der Prüfeinrichtung (9) und der Transporteinrichtung (5,6,7) bzw. der Prüfeinrichtungen und Transporteinrichtungen angeordnet sind.

6. Vorrichtung nach einem der Ansprüche 1 bis 5, **dadurch gekennzeichnet, dass** sämtliche in den Prüfraum (15) hinein- oder aus demselben herausführenden führenden Leitungen und/oder Antriebswellen (6c,7a) spritzwasserdicht oder wasser- und staubdicht durch die Schutzhaube (1) oder den Boden (4) durchgeführt sind.

7. Vorrichtung nach Anspruch 6, **dadurch gekennzeichnet,**
**dass** der Boden (4) mindestens einen Durchbruch, insbesondere zur Durchführung von Leitungen oder einer Antriebswelle (6c,7a), aufweist, welcher mittels einer spritzwasserdichten oder einer wasser- und staubdichten Dichtung abgedichtet ist.

8. Vorrichtung nach Anspruch 3 oder 7, **dadurch gekennzeichnet,**
**dass** die Dichtungen aufblasbare O-Ringdichtungen sind.

9. Vorrichtung nach einem der vorherigen Ansprüche, **dadurch gekennzeichnet,**
**dass** zur Gewährleistung der Spritzwasserdichtheit oder Wasserdichtheit des Prüfraumes (15) sämtliche Zulauf- oder Zuführöffnungen (12) in den Prüfraum (15) und sämtliche Ablauf- oder Abführöffnungen (12) aus dem Prüfraum (15) heraus hermetisch verschließbar sind.

10. Vorrichtung nach einem der Ansprüche 1 bis 9, **dadurch gekennzeichnet,**
**dass** der Boden (4) des Prüfraumes (15) zugleich eine Wandung des Gehäuses (2) ist.

11. Vorrichtung nach einem der Ansprüche 1 bis 10, **dadurch gekennzeichnet,**
**dass** innerhalb des Gehäuses (2) Einrichtungen zum Antrieb, zur Steuerung und zur Versorgung der Prüfeinrichtungen (9) und Transporteinrichtungen (5,6,7), wie Antriebsaggregate, Elektromotoren, elektrische Steuerungen, Signalverstärker, sowie elektrische und/oder optische und/oder mechanische Anschlüsse für die Energieversorgung der Prüfeinrichtung (9), angeordnet sind.

12. Vorrichtung nach einem der Ansprüche 1 bis 11, **dadurch gekennzeichnet,**
**dass** an dem Gehäuse (2), vorzugsweise an dessen Vorderwandung (19), eine von außerhalb des Gehäuses (2) zugängliche Schnittstelle (22) angeordnet ist, in welche eine Mehrzahl von aus dem Gehäuse (2) heraus- oder in dasselbe hineinführenden elektrischen Leitungen mündet und welche zum elektrischen Anschluss der Vorrichtung (7) und/oder zur Übertragung von elektrischen Signalen und/oder Daten zwischen der Vorrichtung (7) und der Außenwelt dient.

13. Vorrichtung nach Anspruch 12, **dadurch gekennzeichnet,**
**dass** an dem Gehäuse (2) eine Schwenktür (3) so angeordnet ist, dass sich zwischen dem Gehäuse (2) und der Schwenktür (3) ein Vorraum (20) befindet.

14. Vorrichtung nach einem der vorherigen Ansprüche, **dadurch gekennzeichnet,**
**dass** die Vorrichtung (17) folgende weiteren Bestandteile umfasst:
- eine innerhalb des Prüfraumes (15) als Teil der Transporteinrichtung (5,6,7) angeordnete Vereinzelungseinrichtung (5), welcher die pharmazeutischen Erzeugnisse aus einem Speicherbehälter zuführbar sind,
- eine über dem Boden (4) des Prüfraumes als weiterer Teil der Transporteinrichtung (5,6,7) angeordnete Fördereinrichtung (7), weiche mindestens eine zum Boden (4) hin offene Aufnahmezelle (8) aufweist, in welche jeweils ein Erzeugnis aus der Vereinzelungseinrichtung (5) einführbar ist, wobei das in der Aufnahmezelle (8) aufgenommene Erzeugnis der Prüfeinrichtung (9) zuführbar ist,
- eine Auswurföffnung (10), welcher das Erzeugnis nach Prüfung durch die Prüfeinrichtung (9) in Fortsetzung des Transportes durch die Fördereinrichtung (7) zuführbar ist, wobei das Erzeugnis nach Erreichen der Auswurföffnung (10) durch diese hindurchfällt und auf diese Weise aus der Aufnahmezelle (8) entfernt wird,
- sowie einen unterhalb der Auswurföffnung (10) angeordneten Sammelbehälter (18) zur Aufnahme der geprüften Erzeugnisse oder deren Teile, nachdem diese durch die Auswurföffnung (10) hindurchgefallen sind.

15. Vorrichtung nach Anspruch 14, **dadurch gekennzeichnet, dass**
- die Transporteinrichtung (7) ein parallel zum Boden (4) angeordneter, um eine vertikale Achse rotierbarer Transportstern (7) ist,
- die Aufnahmezelle (8) im Bereich des Außenumfangs des Transportsterns (7) angeordnet ist,
- und die Prüfeinrichtung (9) sowie die Auswurföffnung (10) so angeordnet sind, dass ein in der Aufnahmezelle (8) aufgenommenes Erzeugnis bei einer Rotation des Transportsterns (7) in dessen Drehrichtung nacheinander zunächst die Prüfeinrichtung (9) und anschließend die Auswurföffnung (10) erreicht.

16. Vorrichtung nach Anspruch 13 und einem der Ansprüche 14 oder 15, **dadurch gekennzeichnet,**
**dass** die Auswurföffnung (10) ein Loch im Boden (4) des Prüfraumes (15) ist und sich der Sammelbehälter (18) in dem Vorraum (20) befindet.

17. Vorrichtung nach einem der Ansprüche 14 bis 16, **dadurch gekennzeichnet,**
**dass** der Boden (4) des Prüfraums um die Auswurföffnung (10) einen Süllrand aufweist.

18. Vorrichtung nach einem der vorhergehenden Ansprüche,
**dadurch gekennzeichnet, dass** die Prüfeinrichtung (9) eine Waage (9) mit einem Waagentisch und einer Kraftmessdose ist.

19. Vorrichtung nach einem der vorherigen Ansprüche, **dadurch gekennzeichnet,**
**dass** das Innere des Gehäuses (2) mit einem Überdruck gegenüber der Umgebung und/oder dem Prüfraum (15) beaufschlagbar ist.

20. Vorrichtung nach einem der vorherigen Ansprüche, **dadurch gekennzeichnet,**
**dass** der Prüfraum (15) mit einem Unterdruck gegenüber der Umgebung beaufschlagbar ist.

21. Vorrichtung nach einem der vorherigen Ansprüche, **dadurch gekennzeichnet,**
**dass** das Gehäuse (2) statt unter dem Boden des Prüfraumes (15) neben demselben angeordnet ist.

22. Vorrichtung nach einem der vorherigen Ansprüche, **dadurch gekennzeichnet,**
**dass** dieselbe Rollen oder Räder aufweist, mittels welchen die Vorrichtung (17) verfahrbar ist.

23. Vorrichtung nach einem der vorherigen Ansprüche, **dadurch gekennzeichnet,**
**dass** die Vorderwandung (19) des Gehäuses (2) aus einem Rahmen (22) und einer auf diesen wasserdicht aufgesetzten abnehmbaren Frontplatte (21) besteht.

## Claims

1. A device (17) for the quality control of solid pharmaceutical products such as tablets, pills, oblongs and the like, comprising at least one testing means (9) having at least one sensor - such as scales (9) and/or a height measuring means and/or a force measuring means for measuring the rupture strength of the product - for checking and ascertaining one or more parameters of the individual pharmaceutical product as well as comprising a transporting means (5, 6, 7) for the products, **characterized in that**
the device (17) has a housing (2) and a test chamber (15) that is arranged above said housing and that can be sealed so as to be splash-proof or water-tight and dust-tight vis-à-vis the environment, said chamber being formed by a floor (4) and by a protective hood (1) that can be placed onto and lifted off this floor, whereby the testing means (9) as well as the transporting means (5, 6, 7) are located in said chamber.

2. The device according to Claim 1, **characterized in that**
the protective hood (1) can be connected in a detachable manner to the floor (4) of the test chamber (15) so as to be splash-proof or water-tight and dust-tight.

3. The device according to Claim 1, **characterized in that**
the protective hood (1) can be pressed onto the periphery of the floor (4) of the test chamber (15) and locked in place so as to be splash-proof or water-tight and dust-tight, whereby the protective hood (1) has an encircling gasket (1b) that is splash-proof or water-tight and dust-tight on its surface (1c) facing the floor (4) of the test chamber (15).

4. The device according to one of Claims 1 to 3, **characterized in that**
the housing (2) can likewise be sealed so as to be splash-proof or water-tight and dust-tight.

5. The device according to one of Claims 1 to 4, **characterized in that**
means for driving, controlling and supplying one or more testing means (9) and one or more transporting means (5, 6, 7) are arranged in the housing (2).

6. The device according to one of Claims 1 to 5, **characterized in that**
all of the lines and/or drive shafts (6c, 7a) leading into or out of the test chamber (15) are configured so as to be splash-proof or water-tight and dust-tight as they pass through the protective hood (1) or the floor (4).

7. The device according to Claim 6, **characterized in that**
the floor (4) has at least one passage, especially for passing lines or a drive shaft (6c, 7a) through, said passage being sealed by means of a gasket that is splash-proof or water-tight and dust-tight.

8. The device according to Claim 3 or 7, **characterized in that**
the gaskets are inflatable O-ring gaskets.

9. The device according to one of the preceding claims, **characterized**
**in that**
all inlet or feed openings (12) leading into the test chamber (15) as well as all outlet or discharge openings (12) leading out of the test chamber (15) can be hermetically sealed so as to ensure that the test chamber (15) is splash-proof or water-tight.

10. The device according to one of Claims 1 to 9, **characterized in that**
the floor (4) of the test chamber (15) is, at the same time, the wall of the housing (2).

11. The device according to one of Claims 1 to 10, **characterized in that**
means for driving, controlling and supplying the testing means (9) and the transporting means (5, 6, 7), such as drive aggregates, electric motors, electric controls, signal amplifiers as well as electric and/or optical and/or mechanical connections to supply energy to the testing means (9) are all arranged inside the housing (2).

12. The device according to one of Claims 1 to 11, **characterized in that**
an interface (22) that is accessible from outside of the housing (2) is arranged on the housing (2), preferably on its front wall (19), whereby a plurality of electric lines leading into or out of the housing (2) open into said interface and the latter serves to establish an electric connection for the device (7) and/or to transmit electric signals and/or data between the device (7) and the outside world.

13. The device according to Claim 12, **characterized in that**
a hinged door (3) is arranged on the housing (2) in such a way as to form an antechamber (20) between the housing (2) and the hinged door (3).

14. The device according to one of the preceding claims, **characterized**
**in that**
the device (17) encompasses the following additional components:
- a singling means (5) as part of the transporting means (5, 6, 7) arranged inside the test chamber (15), whereby the pharmaceutical products can be fed from the storage container to the singling means;
- a conveying means (7) as another part of the transporting means (5, 6, 7) that is arranged above the test chamber floor (4) and that has at least one receiving cell (8) open towards the floor (4) into which one product from the singling means (5) can be introduced, whereby the product accommodated in the receiving cell (8) can be fed to the testing means (9);
- a discharge opening (10) to which the product - after it has been tested by the testing means (9) - can be fed during the further course of transportation by the conveying means (7), whereby the product reaches the discharge opening (10) and then falls through it, as a result of which it is removed from the receiving cell (8);
- as well as a collecting container (18) situated below the discharge opening (10) that serves to receive the tested products or parts thereof once they have fallen through the discharge opening (10).

15. The device according to Claim 14, **characterized in that**
- the transporting means (7) is a star feeder (7) that is arranged parallel to the floor (4) and that can rotate around a vertical axis,
- the receiving cell (8) is situated in the vicinity of the outer circumference of the star feeder (7),
- and the testing means (9) as well as the discharge opening (10) are arranged in such a way that, as the star feeder (7) turns in its direction of rotation, each product accommodated inside the receiving cell (8) first reaches the testing means (9) and subsequently the discharge opening (10).

16. The device according to Claim 13 and one of Claims 14 or 15,
**characterized in that**
the discharge opening (10) is a hole in the floor (4) of the test chamber (15), and the collecting container (18) is located in the antechamber (20).

17. The device according to one of Claims 14 to 16, **characterized in that**
the test chamber floor (4) has a rim around the discharge opening (10).

18. The device according to one of the preceding claims, **characterized**
**in that** the testing means (9) is a set of scales (9) equipped with a weighing platform and a load cell.

19. The device according to one of the preceding claims, **characterized in that** the inside of the housing (2) can be charged with positive pressure relative to the environment and/or to the test chamber (15).

20. The device according to one of the preceding claims, **characterized in that** the test chamber (15) can be charged with negative pressure relative to the environment.

21. The device according to one of the preceding claims, **characterized in that** the housing (2) is situated next to the floor of the test chamber (15) rather than below it.

22. The device according to one of the preceding claims, **characterized in that** the device has rollers or wheels on which the device (17) can be moved.

23. The device according to one of the preceding claims, **characterized**
**in that**
the front wall (19) of the housing (2) consists of a frame (22) and of a removable front plate (21) that can be placed onto the frame so as to be water-tight.

## Revendications

1. Installation (17) pour contrôler la qualité de produits pharmaceutiques solides, tels que des comprimés, des pilules, des oblongs ou des produits de la sorte, comportant au moins un dispositif de contrôle (9), lequel présente au moins un capteur pour le contrôle et la saisie d'un ou de plusieurs paramètres de chaque produit pharmaceutique individuel, tel une balance (9) et/ou un dispositif de mesure de la hauteur et/ou un dispositif dynamométrique pour la mesure de la force d'éclatement du produit, et comportant aussi un dispositif de transport (5,6,7) pour les produits,
**caractérisée en ce que**
l'installation (17) présente un boîtier (2) et une chambre de contrôle (15) disposée au-dessus fermant vis-à-vis de l'environnement ambiant de façon étanche aux projections d'eau ou étanche à l'eau et aux poussières et constituée par un plancher (4) et par un capot de protection (1) qui peut être placé au-dessus et peut être enlevé et dans lequel se trouvent le dispositif de contrôle (9) ainsi que le dispositif de transport (5,6,7).

2. Installation selon la revendication 1, **caractérisée en ce que**
le capot de protection (1) peut être relié au plancher (4) de la chambre de contrôle (15) de façon amovible et étanche aux projections d'eau ou étanche à l'eau et aux poussières.

3. Installation selon la revendication 1, **caractérisée en ce que**
le capot de protection (1) peut être mis en place par pression et verrouillé de façon étanche aux projections d'eau ou étanche à l'eau et aux poussières en périphérie sur le plancher (4) de la chambre de contrôle (15), le capot de protection (1) présentant sur sa face avant (1c) tournée vers le plancher (4) de la chambre de contrôle (15) une garniture (1 b) étanche aux projections d'eau ou étanche à l'eau et aux poussières qui fait le pourtour.

4. Installation selon l'une des revendications 1 à 3, **caractérisée en ce que**
le boîtier (2) peut être également fermé de façon étanche aux projections d'eau ou étanche à l'eau et aux poussières.

5. Installation selon l'une des revendications 1 à 4, **caractérisée en ce que**
des moyens pour l'entraînement, la commande et l'alimentation du dispositif de contrôle (9) et du dispositif de transport (5,6,7) ou des dispositifs de contrôle et des dispositifs de transport sont disposés dans le boîtier (2).

6. Installation selon l'une des revendications 1 à 5, **caractérisée en ce que**
toutes les conduites et/ou arbres d'entraînement (6c, 7a) qui entrent dans la chambre de contrôle (15) ou qui sortent de celle-ci sont conduits à travers le capot de protection (1) ou à travers le plancher (4) de façon étanche aux projections d'eau ou étanche à l'eau et aux poussières.

7. Installation selon la revendication 6, **caractérisée en ce que**
le plancher (4) présente au moins une percée, notamment pour le passage de conduites ou d'un arbre d'entraînement (6c, 7a), laquelle est rendue étanche au moyen d'un joint étanche aux projections d'eau ou étanche à l'eau et aux poussières.

8. Installation selon l'une des revendications 3 ou 7, **caractérisée en ce que**
les garnitures d'étanchéité sont des joints toriques gonflables.

9. Installation selon l'une des revendications précédentes,
**caractérisée en ce que** pour assurer l'étanchéité aux projections d'eau ou l'étanchéité à l'eau de la chambre de contrôle (15), toutes les ouvertures d'amenée ou d'alimentation (12) dans la chambre de contrôle (15) et toutes les ouvertures de sortie ou d'évacuation (12) hors de la chambre de contrôle (15) peuvent être hermétiquement fermées.

10. Installation selon l'une des revendications 1 à 9, **caractérisée en ce que**
le plancher (4) de la chambre de contrôle (15) est en même temps une paroi du boîtier (2).

11. Installation selon l'une des revendications 1 à 10, **caractérisée en ce que**
des moyens pour l'entraînement, la commande et l'alimentation des dispositifs de contrôle (9) et des dispositifs de transport (5,6,7), tels des ensembles d'entraînement, des moteurs électriques, des commandes électriques, des amplificateurs de signaux, ainsi que des raccordements électriques et/ou optiques et/ou mécaniques pour l'alimentation en énergie du dispositif de contrôle (9) sont disposés à l'intérieur du boîtier (2).

12. Installation selon l'une des revendications 1 à 11, **caractérisée en ce que**
sur le boîtier (2), de préférence sur sa paroi avant (19) est disposée une interface (22) accessible de l'extérieur du boîtier (2) dans laquelle aboutissent une pluralité de conduites électriques qui sortent du boîtier (2) ou conduisent dans celui-ci et qui sert au raccordement électrique du dispositif (7) et/ou à la transmission de signaux électriques et/ou de données entre le dispositif (7) et le monde extérieur.

13. Installation selon la revendication 12, **caractérisée en ce que**
une porte à battant pivotant (3) est disposée sur le boîtier (2) de manière à ce qu'une antichambre (20) soit constituée entre le boîtier (2) et la porte à battant pivotant (3).

14. Installation selon l'une des revendications précédentes,
**caractérisée en ce que**
l'installation (17) comprend en outre les éléments suivants :
- un dispositif de distribution des produits un par un (5) disposé à l'intérieur de la chambre de contrôle (15) en tant que partie du dispositif de transport (5,6,7) et auquel les produits pharmaceutiques peuvent être amenés à partir d'un réservoir de stockage,
- un dispositif d'acheminement (7) disposé au-dessus du plancher (4) de la chambre de contrôle en tant qu'autre partie du dispositif de transport (5,6,7), et qui présente au moins une cellule réceptrice (8) ouverte en direction du plancher (4) dans laquelle peut être introduit respectivement chaque produit provenant du dispositif de distribution un par un (5), le produit reçu dans la cellule réceptrice (8) pouvant être amené dans le dispositif de contrôle (9),
- une ouverture d'éjection (10) à laquelle le produit peut être amené après le contrôle par le dispositif de contrôle (9) suite au transport par le dispositif d'acheminement (7), le produit après avoir atteint l'ouverture d'éjection tombant à travers celle-ci et étant de cette manière éliminé de la cellule réceptrice (8),
- ainsi qu'un réservoir collecteur (18) disposé au-dessous de l'ouverture d'éjection (10) pour receuillir les produits contrôlés ou des parties de ceux-ci après que ceux-ci ou celles-ci sont tombé(e)s à travers l'ouverture d'éjection (10).

15. Installation selon la revendication 14, **caractérisée en ce que**
- le dispositif de transport (7) est un dispositif de transport en étoile (7) disposé parallèlement au plancher (4) et pouvant tourner autour d'un axe vertical,
- la cellule réceptrice (8) est disposée dans la zone du pourtour du dispositif de transport en étoile (7),
- et le dispositif de contrôle (9) ainsi que l'ouverture d'éjection (10) sont disposés de sorte que, lors de la rotation du dispositif de transport en étoile (7), le produit recueilli dans la cellule réceptrice (8) atteint tout d'abord le dispositif de contrôle (9) et ensuite l'ouverture d'éjection (10).

16. Installation selon la revendication 13 et l'une ou l'autre des revendications 14 ou 15, **caractérisée en ce que**
l'ouverture d'éjection (10) est un trou dans le plancher (4) de la chambre de contrôle (15) et le réservoir collecteur (18) se trouve dans l'antichambre (20).

17. Installation selon l'une des revendications 14 à 16,
**caractérisée en ce que** le plancher (4) de la chambre de contrôle présente un surbau entourant l'ouverture d'éjection (10).

18. Installation selon l'une des revendications précédentes,
**caractérisée en ce que** le dispositif de contrôle (9) est une balance (9) avec une table de pesage et une boîte dynamométrique.

19. Installation selon l'une des revendications précédentes,
**caractérisée en ce que** l'intérieur du boîtier (2) peut être mis en surpression par rapport au milieu ambiant et/ou à la chambre de contrôle (15).

20. Installation selon l'une des revendications précédentes,
**caractérisée en ce que** la chambre de contrôle (15) peut être mise en dépression par rapport au milieu ambiant.

21. Installation selon l'une des revendications précédentes,
**caractérisée en ce que** le boîtier (2), au lieu d'être disposé au-dessous du plancher de la chambre de contrôle (15), est à côté de celui-ci.

22. Installation selon l'une des revendications précédentes,
**caractérisée en ce que** ladite installation (17) présente des galets ou des roues au moyen desquels elle peut être déplacée.

23. Installation selon l'une des revendications précédentes,
**caractérisée en ce que** la paroi avant (19) du boîtier (2) consiste en un cadre (22) et en une plaque frontale (21) déposée sur celui-ci de façon étanche à l'eau et qui peut être enlevée.
